# EUROPEAN PATENT SPECIFICATION

(11) **EP 2 828 402 B1**
(45) Date of publication and mention of the grant of the patent: **05.07.2017**
(21) Application number: 13713389.8
(22) Date of filing: 20.03.2013
(51) Int. Cl.: C12Q 1/68

(54) **DEVICE FOR DETECTION OF ANALYTES IN AFFINITY BIOASSAYS**
VORRICHTUNG ZUR DETEKTION VON ANALYTEN IN AFFINITÄTS-BIOASSAYS
DISPOSITIF POUR LA DÉTECTION D'ANALYTES DANS DES DOSAGES BIOLOGIQUES PAR AFFINITÉ

(30) Priority: 21.03.2012 EP 12382104
(43) Date of publication of application: 28.01.2015
(73) Proprietor: Vircell, S.L., 18320 Santa Fe (Granada) (ES)
(72) Inventor: MENDOZA MONTERO, Joaquín, E-18008 Granada (ES); ROJAS GONZÁLEZ, Almudena, E-18008 Granada (ES); MENDOZA LÓPEZ, Pablo, E-18008 Granada (ES)
(74) Representative: ZBM Patents - Zea, Barlocci & Markvardsen
(86) International application number: PCT/EP2013/055770
(87) International publication number: WO 2013/139829

(56) References cited:
- WO-A1-00/12675
- US-A1- 2009 181 388

## Description

The present invention relates to devices for analysing a fluid sample and more particular to devices for detection of analytes in affinity bioassays. The invention further relates to the use of such devices for detection of analytes of a nucleic acid amplification reaction such as Polymerase Chain Reaction (PCR).

Furthermore, the invention relates to detection kits comprising such devices and a test strip.

### BACKGROUND ART

The analysis of diagnostically relevant biological samples for the detection of infectious pathogens is gaining importance in the recent years. One of the most widely used methods for pathogen identification involves the use of the Polymerase Chain Reaction (PCR) for the amplification of desired nucleic acid fragments that may subsequently be detected using any known assay.

PCR amplification is very well-known for its speed, ease of use, sensitivity and robustness, since poor quality samples may also be analysed. The PCR amplification of specific target nucleic acids, e.g. a gene or a gene fragment of a particular pathogen, is usually followed by detection of the amplified products, for example by means of an affinity/binding bioassay.

By affinity or binding bioassay it is meant a type of assay to detect bioanalytes by specific interaction of a binding moiety with the target bioanalyte, such binding moieties being molecules or molecule segments capable of binding specifically to the target bioanalyte. The nature of the bioanalyte often determines the binding molecule to be used in its detection. Affinity or binding bioassays usually use a solid support wherein the binding moiety is deposited. These binding/affinity assays are well-known assays used in biotechnology.

A well-known drawback of the diagnosis of infectious pathogens by means of PCR is that contamination may easily occur thus leading to false positives. It is known that one of the main causes of false-positive results relates to contamination of the PCR amplification products due to their manipulation prior to or upon detection. It is thus desirable to reduce such a contamination risk.

A degree of reduction of false positives has been obtained by carrying out PCR reactions in closed environments combining the amplification and detection processes. Document US2010291668, for example, discloses such a device. Other systems may comprise an element that allows attachment to the reaction tube, once the PCR is completed, wherein said element and the reaction tube may be connected to each other in a sealed manner.

Furthermore, portable detection devices are also being developed, which maintain the sample in a relatively closed environment, are relatively easy to handle and are relatively inexpensive. EP2048245 and US20090181388, for example, disclose such a device, but this kind of solution can be relatively cumbersome to operate and therefore the detection process may be expensive to automate. Furthermore, such devices are not quite versatile when more than one amplified sample is to be detected, especially at the same time.

Therefore a need exists to provide a device for detection of analytes, and in particular PCR amplification products, in affinity bioassays which is simple to operate and cost-effective while avoiding contamination, and which also allows an easy automation of the detection process.

### SUMMARY

The present invention generally relates to a device for detection of analytes in affinity bioassays as defined in claim 1. The device comprises at least one transparent wall portion; at least one test recess adapted to receive at least one test strip and arranged such that said test strip is visible through the transparent wall portion; at least one cavity adapted to receive a container containing a fluid sample, said cavity being in fluid communication with said at least one test recess; and piercing means arranged in correspondence with said at least one cavity, such that by pressing down a container into the cavity, towards the end of the pressing down movement, a bottom portion of the container abuts against the piercing means, whereby the container is pierced and at least a portion of the sample is released from the container and reaches the test recess, wherein the transparent wall portion is arranged on a side of the device which is opposite to that in which the at least one cavity is provided; wherein the presence of said transparent wall portion through which the test strip is visible allows reading the results from outside the device. The device works as a substantially enclosed detection unit in combination with a test strip for detection of the analytes, e.g. analytes of an amplified nucleic acid. Contamination of the sample may be avoided because the container is pierced only once it is inside the device, and the sample is then released on the test strip. Contamination of the sample may further be avoided because inserting the container in the device does not involve opening the device. The device may be made in one piece and when it is made from various pieces it may actually be sealed. The device does not require any special arrangement or manipulation but only the insertion of the container with the sample in the cavity. The container is pressed into the cavity, and towards the end of the movement, the bottom portion of the container abuts against the blade or other piercing means, and is therefore pierced. This means that it is the same movement of introduction of the container into the cavity, i.e. the movement to press the container down into the cavity that causes the perforation of the container. This is a direct consequence of arranging the piercing means in correspondence with the cavity such that upon insertion of the container in the cavity the container is pierced. This way, it is rather simple to operate and the detection process may be easily automated as the only action that needs automation is in one direction, the direction of insertion of the container with the sample into the device or what it is the same, the direction of pressing down the container into the cavity. It is thus also a cost-effective solution.

Furthermore, the presence of a transparent wall portion through which the test strip is visible allows reading the results from outside the device. The transparent wall portion is arranged on a side of the device opposite to that in which the at least one cavity may be provided. This way, the device can be easily placed e.g. on a thermic block (thermoblock), whereby the temperature can be relatively easily controlled. This may be of particular interest in implementations in which the detection reaction is a hybridization of DNA wherein the control of temperature may be important.

Moreover, in some implementations in which the test strip may comprise a great number of lines or alternatively a dot-array, the transparent wall portion may be easily placed on a scanner for automatic interpretation of the results.

In some embodiments, the device may further comprise at least one opening provided in a wall of the test recess, opposite to the transparent wall portion. The at least one opening may be arranged to allow fluid communication between the test recess and an internal hollow space of the device. Such openings enhance test strip reading as any evaporation of the released sample may pass through the openings to the internal hollow space and expand, instead of condensing on the test recess' transparent wall portion. These embodiments are especially suitable for reactions taking place at high temperature such as for example DNA-DNA hybridization.

In some embodiments, the device may comprise two or more cavities adapted to receive a container containing a sample. This way, two or more samples, e.g. samples of PCR amplification products, can be detected at the same time and on the same test strip. In some of these cases, the detection reaction may be a hybridization of DNA and the test strip may be provided with dried chemical reagents used for the hybridization reaction. These kinds of assays, called "multitest assays", are of special interest in those cases in which it would be desirable to detect more than one PCR amplification products as mixing various PCR amplification products may go, at least in part, against its efficiency. In alternative embodiments, the further cavities may be used for reagents which may help with the detection reaction. This way, upon release of the fluids held in the containers that may be inserted in the cavities, the fluids may contact the test strip simultaneously or in a stepwise manner. These reagents may be used e.g. for improving reaction performance. For example, the use of a stringent wash solution may improve specificity of the test, reducing background signal. In another example, conjugate or developing reagents may be added using the second (or third) cavities after introducing the first container with the sample in the first cavity.

In some of these embodiments comprising two or more cavities, at least two cavities may be arranged at an angle and they may be converged towards the same area of the test recess. This way, the fluids contained in each container inserted in each cavity may react simultaneously on a test strip provided in the test recess. In other embodiments, at least two cavities may be arranged at an angle and they may be directed towards different areas of the test recess. This way, the reactions of the fluids contained in the containers inserted in these cavities may be consecutive. This alternative is especially interesting in those cases wherein the further cavities contain reagents.

In some embodiments, at least one cavity may comprise a bulge portion arranged on a wall of the cavity such that when the container containing a sample is inserted in the cavity, the bulge portion generates a pressure increment inside the container. Such a pressure increment inside the container ensures the release of sample out of the container and towards the test recess once the container is introduced into the cavity and is thereby pierced.

In some embodiments, the device may be a single-use disposable device and the discarded unit may be left in a safety place.

Another aspect relates to the use of a device substantially as hereinbefore described for detection of analytes of a nucleic acid amplification reaction.

In a further aspect a detection kit comprising a device substantially as hereinbefore described and a test strip is provided. The test strip may have at least a test area comprising dried chemical reagents used for affinity bioassays.

In some embodiments, the test strip may further comprise a control area and the test area may comprise a pad having the dried chemical reagents used for hybridization reaction. In others, it may also further comprise a control area and the test area may comprise dried chemical reagents used for an immunoassay.

Additional objects, advantages and features of embodiments of the invention will become apparent to those skilled in the art upon examination of the description, or may be learned by practice of the invention.

### BRIEF DESCRIPTION OF THE DRAWINGS

Particular embodiments of the present invention will be described in the following by way of non-limiting examples, with reference to the appended drawings, in which:
Figure 1 shows an exploded view of a device according to a first embodiment;
Figure 2 shows a side view of the device of figure 1;
Figure 3 shows a perspective view of the device of figure 1;
Figure 4 shows a top view of a device according to a second embodiment;
Figure 5 shows an exploded view of the device of figure 4;
Figure 6a and 6b show respectively a front and a side view of the device of figure 4;
Figure 7 shows bottom view of the device of figure 4; and
Figures 8a to 8f show a further embodiment.

### DETAILED DESCRIPTION OF EMBODIMENTS

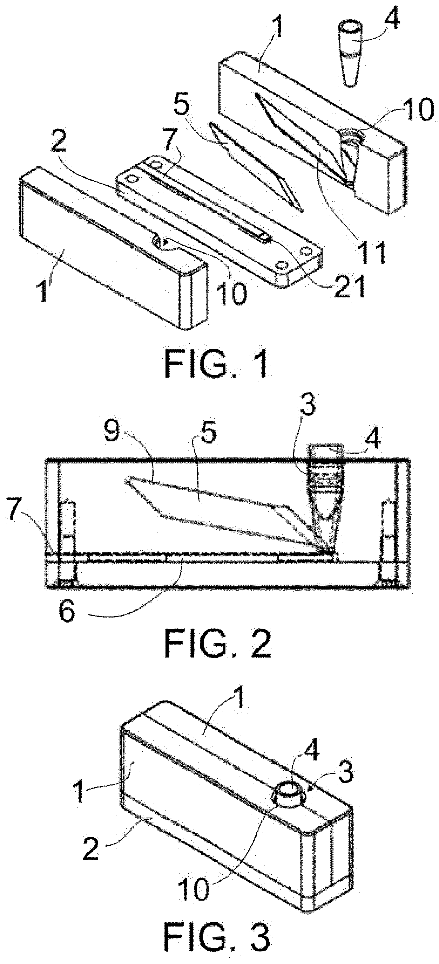

Figure 1 shows an exploded view of a device according to a first embodiment. Figure 1 shows a device that may be made from two first bodies 1 and a cover 2. Each first body 1 may have a first concavity 10 and a second concavity 11. The two first bodies 1 may be joined together with their concavities 10 and concavities 11 matching together respectively (see figure 3). This way a cavity (see figure 3) may be defined by the two first concavities 10 and a housing 9 (see figure 2) may be defined by the two second concavities 11. In such a housing 9 piercing means, e.g. a blade 5, may be arranged.

Moreover the second concavities 11 may be arranged in correspondence with the first concavities 10 such that the blade 5 arranged in the housing 9 may project into the cavity 3 and may pierce a container 4 when it is inserted therein. This way, with a single action or movement, i.e. inserting the container into the cavity, the container is at the same time placed in the cavity and pierced, and the sample may thus be released. Furthermore, such a single movement may be done in only one direction: the pressing down direction. This means that the device is rather simple to operate and that the detection process, including the introduction and piercing of containers, can easily be automated.

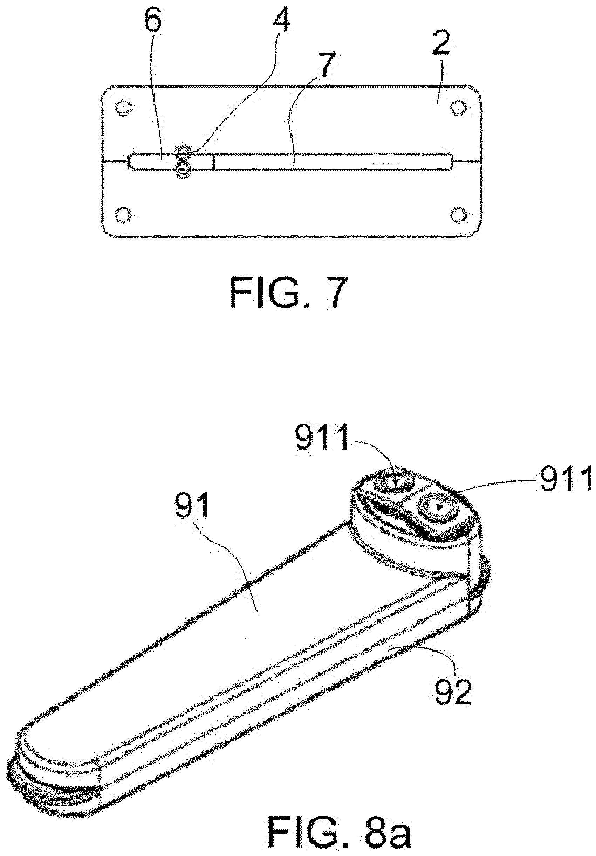

Furthermore, figure 1 shows that the cover 2, which may be a transparent cover, may comprise a third concavity (see arrow 21). When the transparent cover 2 is mounted together with the two first bodies 1, such a third concavity 21 may define a test recess (see figure 2) in fluid communication with the cavity 3 defined by the two first concavities 10. A test strip 7 may be arranged in the test recess. The test strip 7 may be arranged such that it may be visible from outside the device through the transparent cover 2. In alternative implementations more than one test strip may be arranged in the test recess. It should be understood that in other implementations other ways of forming the test recess may be foreseen, e.g. protrusions from the cover as shown in the example of figures 8a-8f.

Figure 2 shows a side view of figure 1 wherein a first body has been made transparent in order to show the interior of the device. Figure 2 shows that the test recess 6 may be in fluid communication with the cavity 3 and that the blade 5 may pierce a bottom portion of a container 4 inserted in the cavity 3. This means that upon insertion of the container 4 in the cavity 3 the container 4 may thereby be pierced and at least a portion of sample is released from the container 4 and reaches a test strip 7 that may be arranged in the test recess 6.

Figure 3 shows a perspective view of figure 1. Figure 3 shows a cavity 3 that may be defined by the two first concavities 10. A container 4 containing a fluid sample may be inserted in the cavity 3.

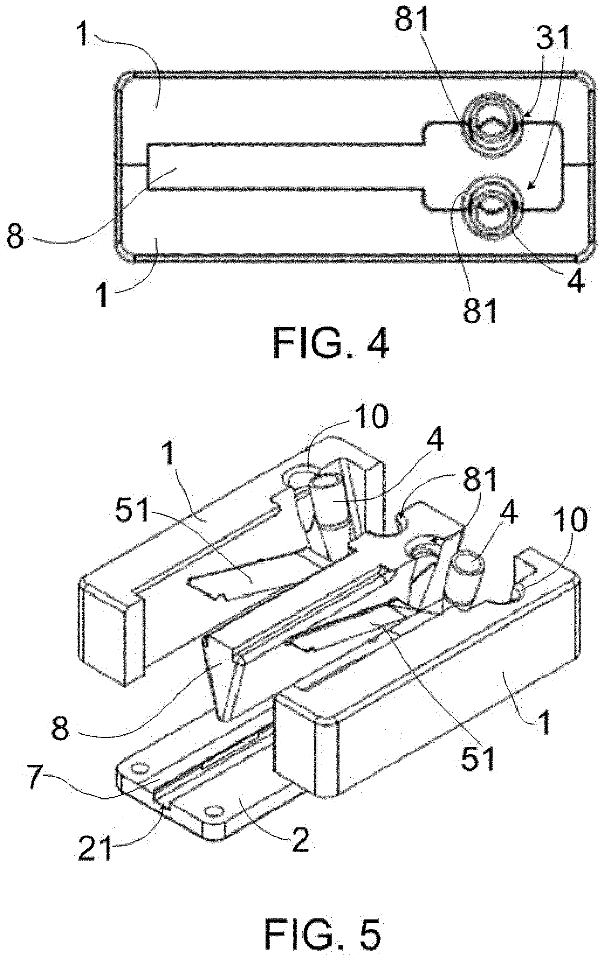

Figure 4 shows a top view of a device according to a second embodiment. The same reference numbers will be used for matching parts. Figure 4 shows a device that may have two cavities 31 adapted to receive a container 4 containing a fluid sample. Differently from the example of figure 1, the example of figure 4 shows a device that may comprise a second body 8 that may be mounted in between the two first bodies 1. Such a second body 8 may have two fourth concavities 81. Each fourth concavity 81 may match with a first concavity of the first bodies 1. This way, when the bodies 1 and 8 are mounted together with the cover (see figure 5), two cavities 31 may be defined instead of only one as in the example of figure 1. It should be understood that in other implementations more concavities may be present defining more cavities. The device may also be made in one piece or from various pieces/bodies substantially as hereinbefore explained.

Figure 5 shows an exploded view of figure 4. Figure 5 shows a second body 8 that may comprise two fourth concavities 81. Each fourth concavity 81 together with each first concavity 10 provided in the two first bodies 1 can define a cavity (See figure 4). Figure 5 shows that the fourth concavities 81 may each be in correspondence with a blade 51 such that when a container 4 is inserted in each cavity 31 a blade 51 projects into each cavity 31 and pierces a bottom portion of each container 4.

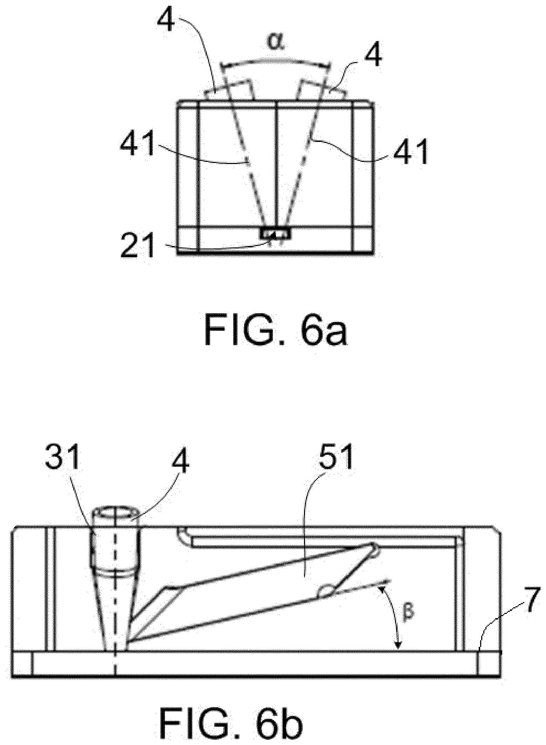

Figure 6a and 6b show a front and a side view of figure 4 respectively wherein the first bodies have been made transparent in order to show the interior of the device.

Figure 6a shows two containers 4 that may be inserted in the two cavities (see figure 4). A symmetry axis 41 of each container 4 shows that when they are inserted in the cavities they may be arranged at an angle α and may be converged towards the same portion of the third concavity 21 provided in the cover 2 wherein a test strip may be arranged. This way, two different samples contained in two different containers 4 inserted in the two cavities may flow towards the same area and may react simultaneously on the test strip 7. In a preferred embodiment, in an example of a device having two cavities, the angle α may be around 30°.

In alternative embodiments, the two cavities may be arranged at an angle and may be directed towards different areas of the test recess wherein a test strip may be arranged. This way, the reactions of the fluids contained in the containers inserted in the cavities may be consecutive and a sample may be reacted with different reagents consecutively.

Figure 6b shows that the blades 51 (only one is shown) are arranged with an inclination β from the horizontal. The blades 51 may thus pierce a bottom portion of a container 4 when it is inserted in each cavity 31. This way, at least a portion of sample is released from the container 4 and reaches a test strip 7 that may be arranged in the test recess. In a preferred embodiment, in an example of a device having two cavities, the angle β may be around 13°.

Figure 7 shows a bottom view of the device of figure 4. The test strip 7 may be visible from outside the device through the transparent cover 2. Furthermore, figure 7 shows the two containers 4 converging towards the same portion of the test recess 6. The cover 2 may be a flat surface with no protuberances therein and it may thus be easily placed on a scanner (not shown) for automatic interpretation of the results. In alternative embodiments, other types of readers may be used, portable or not, or the scanner may be combined with a computer which may provide quantitative or qualitative results.

Furthermore, the cover may be easily placed e.g. on a thermic block (thermoblock), whereby the temperature can be relatively easily controlled. This may be of particular interest in implementations in which the detection reaction is a hybridization of DNA wherein the control of temperature may be important.

In some of above embodiments, the bioanalyte to be detected may be a DNA or a RNA molecule. In some of these cases it may be the amplification product of a PCR reaction. In this case, the binding molecule may often be a hybridization probe, i.e. a fragment of DNA or RNA of variable length (usually 100-1000 bases long), which may detect the presence of a target sequence that is complementary to the sequence in the probe. The probe thereby hybridizes to nucleic acid (DNA or RNA) whose base sequence allows probe-target base pairing due to complementarity between the probe and the target.

In others, the bioanalyte to be detected may be a protein and the binding molecule to be used can thus be an antibody. Affinity bioassays that use antibodies or molecules that have antibody-like functions, such as aptamers, are generally called immunoassays. A typical example of an immunoassay is an Enzyme-Linked ImmunoSorbent Assay (ELISA).

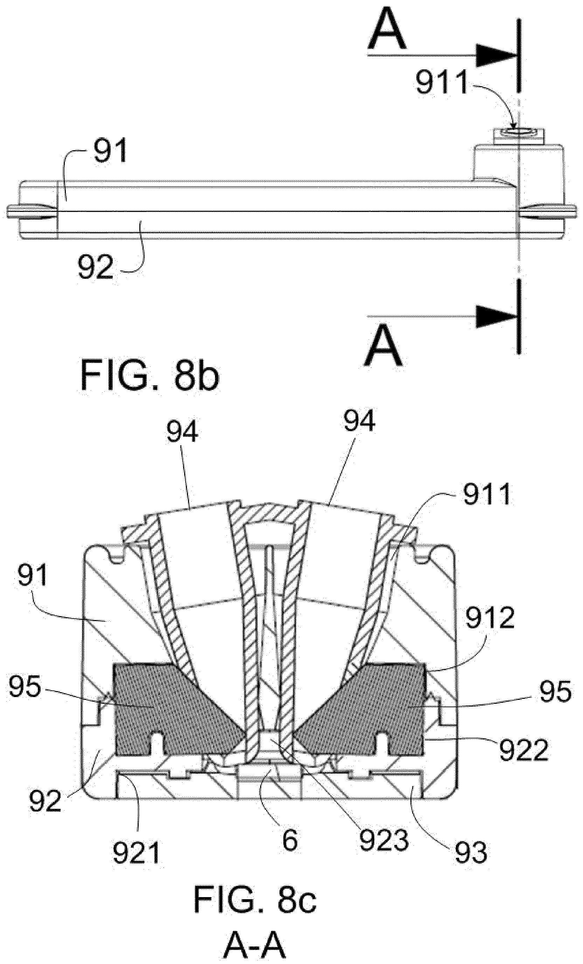

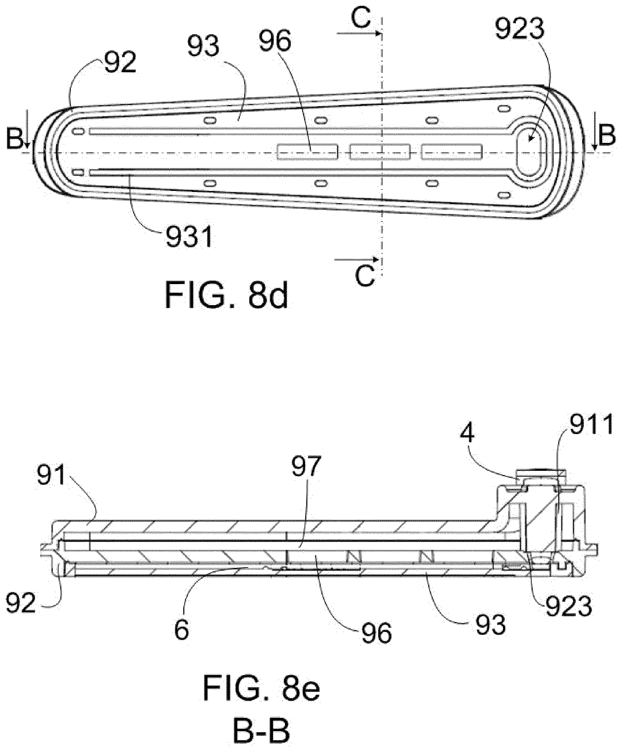

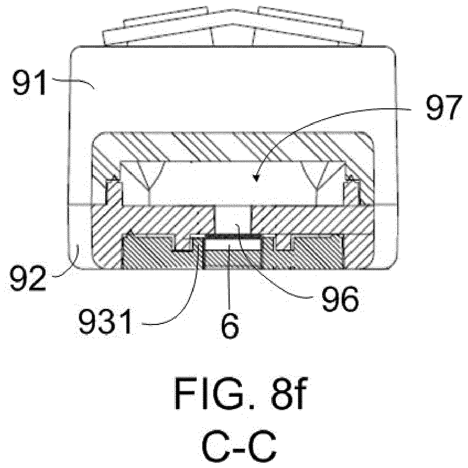

Figure 8a shows a perspective view a device according to a further embodiment. Figure 8b shows a side view, figure 8c a cross-sectional view along line A-A of figure 8b, figure 8d shows a bottom view of the device, figure 8e shows a longitudinal section along line B-B of figure 8d and figure 8f shows a cross-section along line C-C of figure 8d. The same reference numbers will be used for matching parts.

Figures 8a-8c, 8e and 8f show a device that may be made from a first body 91 and a second body 92. The first body 91 may be arranged in a substantially stacked manner on top of the second body 92. The first body 91 may comprise two cavities 911 adapted to receive each a container 4 containing a fluid sample.

Figure 8d, shows that the second body 92 may further comprise an orifice 923 in correspondence with the cavities 911 of the first body 91 to allow container insertion and fluid communication between the cavities 911 and a test recess 6 may be defined by the second body 92 and a transparent cover 93.

As shown in figure 8c, the first body 91 and second body 92 may each comprise one or more indents 912 and 922 for housing one or more blades 95. The blades 95 may further project into the cavities 911 and the orifice 923 thereby piercing a container 4 when it is pressed down for insertion.

Figure 8c further shows that the transparent cover 93 may be housed in a concavity 921 of the second body 92. As shown in figure 8d the transparent cover 93 may allow a view from the bottom of the second body 92 when there is no test strip housed in the test recess 6 and when there is a test strip (not shown) housed in the test recess, it may be readable from the outside through the transparent cover. The test recess 6 may be made e.g. from a continuous protrusion 931 (see also figure 8f) of the transparent cover 93.

Figures 8d to 8f show that openings 96 may be arranged in a wall of the test recess 6, opposite to the transparent cover 93, for reducing condensation inside the test recess 6. Furthermore, the openings 96 may be arranged to allow fluid communication between the test recess 6 and an internal hollow space 97 of the device provided between the first body 91 and the second body 92 (see fig. 8f). This way, any evaporation of the released sample may pass through the openings 96 to the internal hollow space 97 and expand, instead of condensing on the test recess' transparent cover 93.

In some embodiments, a volume of the internal hollow space may be larger than that of the test recess. This improves the passage of sample evaporation towards the internal hollow space.

In figures 8d and 8e only three openings 96 are shown, but other number may also be possible and even only one opening is possible.

The embodiment represented in figures 8a-8f is especially suitable for reactions taking place at elevated temperature, i.e. 37-60 °C, for example, for the detection of products of nucleic acid amplification (PCR, SDA, TMA) by detecting the reaction of DNA-DNA hybridization (not hapten-antihapten).

Although two cavities are present in the example shown in figures 8a-8f, it should be understood that in other implementations, other number of cavities may also be foreseen and even only one cavity may be provided. The device may also be made in one piece or from various pieces/bodies substantially as hereinbefore explained.

In some embodiments, a device substantially as hereinbefore described may further comprise a bulge portion arranged on a wall of the at least one cavity. This way, when the container containing sample is inserted in the cavity the bulge portion may generate a pressure increment inside the container. The size of the at least one cavity may depend on the size of the container containing the sample that will be used. This way, some pressure may have to be exerted on the container in order to fit it in the at least one cavity.

In some embodiments, the container containing a sample may be any closed container such as micro centrifuge tubes e.g. a container with a conical bottom and an integral snap cap usually known as Eppendorf. An advantage of a device substantially as hereinbefore described used in combination with a container such as an Eppendorf relates to avoidance of the need to open the container with the sample after e.g. the amplification reaction is carried out and prior to or upon detection. The device only needs the Eppendorf containing the amplified sample to be inserted in the at least one cavity. This minimizes the chances of contamination of the sample and, therefore, false positives in the analysis.

In some embodiments, the test strip may be replaced by any support having binding elements such as a substrate having a surface containing one or more synthetic capture units capable of specifically binding with a synthetic binding unit of a target analyte. For example, the substrate may include a plurality of synthetic capture units that are positioned on the substrate so as to form one or more addressable lines. Such a substrate may be placed on the test recess of the device so as to avoid movement of the substrate, though it does not necessarily need to be encased within the test recess. A variety of materials may be used as the substrate; such materials are known to those skilled in the art.

In some embodiments, the device may further comprise a sealing material arranged on part of its outer surface or in between the different bodies forming the device. This may help to ensure its closure.

## Claims

1. A device for detection of analytes in affinity bioassays comprising:
- at least one transparent wall portion (2; 93),
- at least one test recess (6) adapted to receive at least one test strip (7) and arranged such that said test strip (7) is visible through the transparent wall portion (2; 93),
- at least one cavity (3; 911) adapted to receive a container (4) containing a fluid sample, said cavity (3; 911) being in fluid communication with said at least one test recess (6), and
- piercing means (5; 95) arranged in correspondence with said at least one cavity (3; 911), such that by pressing down a container (4) into the cavity (3; 911), towards the end of the pressing down movement, a bottom portion of the container (4) abuts against the piercing means (5; 95), whereby the container (4) is pierced and at least a portion of the sample is released from the container (4) and reaches the test recess (6), wherein the transparent wall portion (2; 93) is arranged on a side of the device which is opposite to that in which the at least one cavity (3; 911) is provided; wherein the presence of said transparent wall portion (2; 93) through which the test strip (7) is visible allows reading the results from outside the device.

2. Device according to claim 1, further comprising one or more openings (96) in a wall of the test recess (6) opposite to the transparent wall portion (93), the openings (96) being arranged to allow fluid communication between the test recess (6) and an internal hollow space (97) of the device.

3. Device according to claim 2, wherein a volume of the internal hollow space (97) is larger than that of the test recess (6).

4. Device according to any of claims 1 - 3, comprising two or more cavities (911) adapted to receive a container containing a sample.

5. Device according to claim 4, wherein at least two cavities (911) are arranged at an angle and are converged towards the same area of the test recess (6).

6. Device according to claim 4, wherein at least two cavities (911) are arranged at an angle and are directed towards different areas of the test recess (6).

7. Device according to any of claims 1-6, wherein at least one cavity (3; 911) comprises a bulge portion arranged on a wall of the cavity such that when the container (4) containing a sample is inserted in the cavity (3; 911), the bulge portion generates a pressure increment inside the container (4).

8. Device according to any of claims 1-7, wherein the piercing means (5; 95) are one or more blades arranged such that they cut a bottom portion of the container (4).

9. Device according to any of claims 1-8 comprising at least two bodies (1, 2; 8) having concavities (10, 11; 81) defining the at least one cavity (3; 31) adapted to receive a container (4) and the at least one recess (9) for housing the piercing means (5; 95).

10. Device according to claim 9, further comprising a transparent cover (2; 93) having a further concavity (21; 921) arranged such that the test recess (6) is defined between the two bodies (1; 92) and the further concavity of the transparent cover.

11. Device according to any of claims 1-10, wherein it is a single-use disposable device.

12. Detection kit comprising a device according to any of claims 1-11 and a test strip having at least a test area comprising dried chemical reagents used for affinity bioassays.

13. Detection kit according to claim 12, wherein the test strip further comprises a control area and the test area comprises a pad having the dried chemical reagents used for hybridization reaction or the test area comprises dried chemical reagents used for an immunoassay.

14. Use of a device according to any of claims 1-11 for detection of analytes of a nucleic acid amplification reaction.

## Patentansprüche

1. Eine Vorrichtung zur Detektion von Analyten in Affinitäts-Bioassays umfassend:
- mindestens einen transparenten Wandteil (2; 93),
- mindestens eine Versuchsaushöhlung (6), die zum Empfang von mindestens einem Versuchsband (7) angepasst ist und derart angeordnet ist, dass das Versuchsband (7) durch den transparenten Wandteil (2; 93) sichtbar ist,
- mindestens eine Vertiefung (3; 911), die angepasst zum Empfang von einem Reservoir (4) ist, das eine Flüssigkeitsprobe enthält, wobei die Vertiefung (3; 911) in Fluidverbindung mit der mindestens einer Versuchsaushöhlung (6) ist, und
- ein Durchstechmittel (5; 95), das entsprechend der mindestens einen Vertiefung (3; 911) angeordnet ist, so dass, wenn das Reservoir (4) in die Vertiefung (3; 911) hinein gedrückt wird, ein unterer Teil des Reservoirs (4) gegen Ende der Herunterdrückbewegung an das Durchstechmittel (5; 95) anstoßt, wodurch das Reservoir (4) durchgestochen wird und mindestens ein Teil der Probe aus dem Reservoir (4) freigesetzt wird und die Versuchsaushöhlung (6) erreicht, wobei der transparente Wandteil (2; 93) auf einer Seite der Vorrichtung angeordnet ist, welche gegenüber dem Teil liegt, in dem die mindestens eine Vertiefung (3; 911) bereitgestellt ist; wobei das Vorhandensein des transparenten Wandteils (2; 93), durch den das Versuchsband (7) sichtbar ist das Ablesen der Ergebnisse von außerhalb der Vorrichtung ermöglicht.

2. Vorrichtung nach Anspruch 1, weiterhin umfassend eine oder mehrere in einer Wand der Versuchsaushöhlung (6) gegenüber dem transparenten Wandteil (93) vorhandene Öffnungen (96), wobei die Öffnungen (96) angeordnet sind, um eine Fluidverbindung zwischen der Versuchsaushöhlung (6) und einem inneren hohlen Raum (97) der Vorrichtung zu gewährleisten.

3. Vorrichtung nach Anspruch 2, wobei ein Volumen des inneren hohlen Raums (97) größer als das der Versuchsaushöhlung (6) ist.

4. Vorrichtung nach einem der Ansprüche 1 - 3, umfassend zwei oder mehrere Vertiefungen (911), die zum Empfang von einem Reservoir angepasst sind, das eine Probe enthält.

5. Vorrichtung nach Anspruch 4, wobei mindestens zwei Vertiefungen (911) schräg angeordnet sind und zum gleichen Bereich der Versuchsaushöhlung (6) konvergieren.

6. Vorrichtung nach Anspruch 4, wobei mindestens zwei Vertiefungen (911) schräg angeordnet sind und nach verschiedenen Bereichen der Versuchsaushöhlung (6) ausgerichtet sind.

7. Vorrichtung nach einem der Ansprüche 1-6, wobei mindestens eine Vertiefung (3; 911) einen gewölbten Teil umfasst, der auf einer Wand der Vertiefung angeordnet ist, so dass, wenn das eine Probe enthaltende Reservoir (4) in die Vertiefung (3; 911) eingeführt wird, der gewölbte Teil eine Druckerhöhung innerhalb des Reservoirs (4) hervorruft.

8. Vorrichtung nach einem der Ansprüche 1-7, wobei das Durchstechmittel (5; 95) in einem oder mehreren Blättern besteht, welche derart angeordnet sind, dass sie einen unteren Teil des Reservoirs (4) schneiden.

9. Vorrichtung nach einem der Ansprüche 1-8 umfassend mindestens zwei Körper (1, 2; 8), welche Innenwölbungen (10, 11; 81) aufweisen, die mindestens eine Vertiefung (3; 31) definieren, welche angepasst zum Empfang von einem Reservoir (4) und von der mindestens einen Aushöhlung (9) zur Aufnahme des Durchstechmittels (5; 95) ist.

10. Vorrichtung nach Anspruch 9, weiterhin umfassend einen transparenten Deckel (2; 93), der eine weitere Innenwölbung (21; 921) aufweist, die so angeordnet ist, dass die Versuchsaushöhlung (6) zwischen den zwei Körpern (1; 92) und der weiteren Innenwölbung des transparenten Deckels definiert ist.

11. Vorrichtung nach einem der Ansprüche 1-10, welche eine EinwegVorrichtung ist.

12. Detektionskit umfassend eine Vorrichtung nach einem der Ansprüche 1-11 und ein Versuchsband, das mindestens einen Versuchsbereich umfassend getrocknete chemische Reagenzien hat, die in Affinitäts-Bioassays verwendet werden.

13. Detektionskit nach Anspruch 12, wobei das Versuchsband weiterhin einen Kontrollbereich umfasst und der Versuchsbereich ein Pad hat, der die getrockneten chemischen Reagenzien umfasst, welche zur Hybridisierungsreaktion verwendet werden oder der Versuchsbereich getrocknete chemische Reagenzien umfasst, die in einem Immunoassay verwendet werden.

14. Verwendung von einer Vorrichtung nach einem der Ansprüche 1-11 zur Detektion von Analyten einer Nukleinsäure-Amplifikationsreaktion.

## Revendications

1. Un dispositif pour la détection d'analytes dans des dosages biologiques par affinité comprenant :
- au moins une partie de paroi transparente (2 ; 93),
- au moins un renfoncement de test (6) adapté pour recevoir au moins une bande de test (7) et disposé de façon que ladite bande de test (7) est visible à travers la partie de paroi transparente (2 ; 93),
- au moins une cavité (3 ; 911) adaptée pour recevoir un réservoir (4) contenant un échantillon de fluide, étant ladite cavité (3 ; 911) en communication fluidique avec ledit au moins un renfoncement de test (6), et
- un moyen de perçage (5 ; 95) disposé en correspondance avec ladite au moins une cavité (3 ; 911), de façon que en pressant un réservoir (4) dans la cavité (3 ; 911), vers la fin du mouvement de pressage, une partie inférieure du réservoir (4) bute contre le moyen de perçage (5 ; 95), par lequel le réservoir (4) est percé et au moins une partie de l'échantillon est libérée du réservoir (4) et atteint le renfoncement de test (6), dans lequel la partie de paroi transparente (2; 93) est disposée sur un côté du dispositif qui est situé en face du côté dans lequel l'au moins une cavité (3; 911) est située ; dans lequel la présence de ladite partie de paroi transparente (2; 93) à travers laquelle la bande de test (7) est visible permet d'effectuer la lecture des résultats de l'extérieur du dispositif.

2. Dispositif selon la revendication 1, comprenant en outre une ou plusieurs ouvertures (96) dans une paroi du renfoncement de test (6) située en face de la partie de paroi transparente (93), étant les ouvertures (96) disposées de façon à permettre la communication fluidique entre le renfoncement de test (6) et un espace creux intérieur (97) du dispositif.

3. Dispositif selon la revendication 2, dans lequel un volume de l'espace creux intérieur (97) est plus grand que celui du renfoncement de test (6).

4. Dispositif selon l'une quelconque des revendications 1 - 3, comprenant deux ou plus cavités (911) adaptées pour recevoir un réservoir contenant un échantillon.

5. Dispositif selon la revendication 4, dans lequel au moins deux cavités (911) sont disposées à un angle et convergent vers la même région du renfoncement de test (6).

6. Dispositif selon la revendication 4, dans lequel au moins deux cavités (911) sont disposées à un angle et orientées vers des régions différentes du renfoncement de test (6).

7. Dispositif selon l'une quelconque des revendications 1-6, dans lequel au moins une cavité (3 ; 911) comprend une partie protubérante disposée sur une paroi de la cavité de façon que lorsque le réservoir (4) contenant un échantillon est introduit dans la cavité (3 ; 911), la partie protubérante génère une augmentation de pression dans le réservoir (4).

8. Dispositif selon l'une quelconque des revendications 1-7, dans lequel le moyen de perçage (5 ; 95) consiste en une ou plusieurs pales disposées de façon qu'elles coupent une partie inférieure du réservoir (4).

9. Dispositif selon l'une quelconque des revendications 1-8 comprenant au moins deux corps (1, 2 ; 8) ayant des concavités (10, 11 ; 81) définissant l'au moins une cavité (3 ; 31) adaptée pour recevoir un réservoir (4) et l'au moins un renfoncement (9) pour loger le moyen de perçage (5 ; 95).

10. Dispositif selon la revendication 9, comprenant en outre un couvercle transparent (2 ; 93) ayant une autre concavité (21 ; 921) disposée de façon que le renfoncement de test (6) est défini entre les deux corps (1 ; 92) et l'autre concavité du couvercle transparent.

11. Dispositif selon l'une quelconque des revendications 1-10, dans lequel il s'agit d'un dispositif jetable à usage unique.

12. Kit de détection comprenant un dispositif selon l'une quelconque des revendications 1-11 et une bande de test ayant au moins une région de test comprenant des réactifs chimiques secs utilisés pour des dosages biologiques par affinité.

13. Kit de détection selon la revendication 12, dans lequel la bande de test comprend en outre une région de contrôle et la région de test comprend un coussinet ayant les réactifs chimiques secs utilisés pour la réaction d'hybridation ou la région de test comprend des réactifs chimiques secs utilisés pour un immunoessai.

14. Utilisation d'un dispositif selon l'une quelconque des revendications 1-11 pour la détection d'analytes d'une réaction d'amplification de l'acide nucléique.
